# EUROPEAN PATENT APPLICATION

(11) **EP 1 686 162 A2**
(43) Date of publication of application: **02.08.2006**
(21) Application number: 06100447.9
(22) Date of filing: 17.01.2006
(51) Int. Cl.: C09J 7/02, A61L 15/58

(54) **Tape Preparation**

(30) Priority: 26.01.2005 JP 2005018816
(71) Applicant: NITTO DENKO CORPORATION, Ibaraki-shi, Osaka 567-8680 (JP)
(72) Inventor: Hanatani, Akinori c/o Nitto Denko Corporation, Osaka, 567-8680 (JP); Ito, Masaaki c/o Nitto Denko Corporation, Osaka, 567-8680 (JP); Saito, Junichi c/o Nitto Denko Corporation, Osaka, 567-8680 (JP)
(74) Representative: Weber, Thomas

(57) **Abstract**

The present invention provides a tape preparation including a support and an adhesive layer directly or indirectly formed on at least one surface of the support, wherein the adhesive layer contains a rubber adhesive containing at least one kind of rubber polymer and capsaicin as an active ingredient. Since the adhesive layer shows extremely fine adhesive property and superior capsaicin releaseability, a rapid pharmacological effect can be afforded with a small capsaicin content.

## Description

### TECHNICAL FIELD OF THE INVENTION

The present invention relates to a tape preparation comprising capsaicin as an active ingredient, which is used for continuous administration of the active ingredient into living organisms.

### BACKGROUND OF THE INVENTION

Continuous administration of capsaicin to living organisms induces selective degeneration of primary sensory neuron and degrades sensation of pain to stimulation (Martin Hautkappe et al. "Review of the Effectiveness of Capsaicin for Painful Cutaneous Disorders and Neural Dysfunction, Clin J Pain 14: 97-106. 1998). As external medicine containing capsaicin, which aims at relieving pain or itching in postherpetic neuralgia, diabetic neuralgia, essential pruritus and the like, cream and ointment utilizing this action have been known (Zostrix, Gen Derm Corporation, U.S.A.), (JP-B-3211027, Japan Hospital Pharmacists Association ed. "Hospital Pharmacists Preparation, Forth Edition" 189. 1997). However, since capsaicin, a pungent component of red pepper, temporarily causes causalgia and stimulant pain (initial stimulant pain), its adhesion to a part other than the affected part is not desirable. Such adhesion can be prevented by employing a tape preparation. To be specific, since an agent containing capsaicin is covered with a support in tape preparations, the risk of contamination of parts other than the object site can be reduced. Moreover, while cream and ointment are generally applied to the affected part with a finger, tape preparations can be applied to the affected part without touching the agent containing capsaicin. Furthermore, when an excruciating pain appears due to capsaicin, tape preparations permit complete and easy removal of an agent containing capsaicin by only peeling off the preparation, which is a merit of tape preparations.

There are a number of reports relating to tape preparations aiming at an analgesic or antipruritic effect (JP-B-3159688), and there are also a number of reports relating to tape preparations containing capsaicin (JP-A-11-209269). They use capsaicin as an auxiliary agent to give warm sensation, facilitate blood circulation and the like, whereas there are only a small number of reports on the above-mentioned tape preparations containing capsaicin as a principal agent, which aim at an analgesic or antipruritic effect based on the physiological activity of capsaicin, and the compositions of such preparations are complicated (JP-T-2002-514221). As mentioned above, there can be found no report on an analgesic or antipruritic tape preparation containing capsaicin, which permits easy production and easy quality control and which is superior in practicality.

### SUMMARY OF THE INVENTION

Accordingly, it is an object of the present invention to provide a tape preparation comprising capsaicin as a principal agent and having an analgesic or antipruritic effect, which permits easy production and easy quality control, and which is superior in practicality.

In an attempt to solve the above-mentioned problems, the present inventors have considered a combination of capsaicin, various adhesives and various additives as components of an adhesive layer of a tape preparation comprising a support and the adhesive layer. As a result, they have found that, when a rubber adhesive and capsaicin as an active ingredient are contained in an adhesive layer of a tape preparation, the release of capsaicin (transdermal transfer) becomes superior and rapid efficacy can be achieved even with a small capsaicin content.

Accordingly, the present invention provides the following.
(1) A tape preparation comprising a support and an adhesive layer directly or indirectly formed on at least one surface of the support, wherein the adhesive layer comprises a rubber adhesive containing at least one kind of rubber polymer and capsaicin as an active ingredient.
(2) The tape preparation of the above-mentioned (1), wherein the rubber adhesive further comprises a tackifier.
(3) The tape preparation of the above-mentioned (1), wherein the rubber adhesive layer further comprises a fatty acid ester.
(4) The tape preparation of any of the above-mentioned (1)-(3), wherein the fatty acid ester is obtained from a higher fatty acid having 10 to 16 carbon atoms and a lower monovalent alcohol having 1 to 4 carbon atoms.
(5) The tape preparation of any of the above-mentioned (1)-(4), which is used for the treatment of pain or itching.
(6) The tape preparation of the above-mentioned (5), wherein the pain or itching is associated with postherpetic neuralgia, diabetic neuralgia, essential pruritus, psoriasis, cluster headache, post-mastectomy pain syndrome, stomatitis, skin allergy, urination reflection facilitation, lumbago in hematuria syndrome, cervical pain, stump pain, reflex sympathetic atrophy, pain due to skin cancer or arthritis.

### BRIEF DESCRIPTION OF THE DRAWING

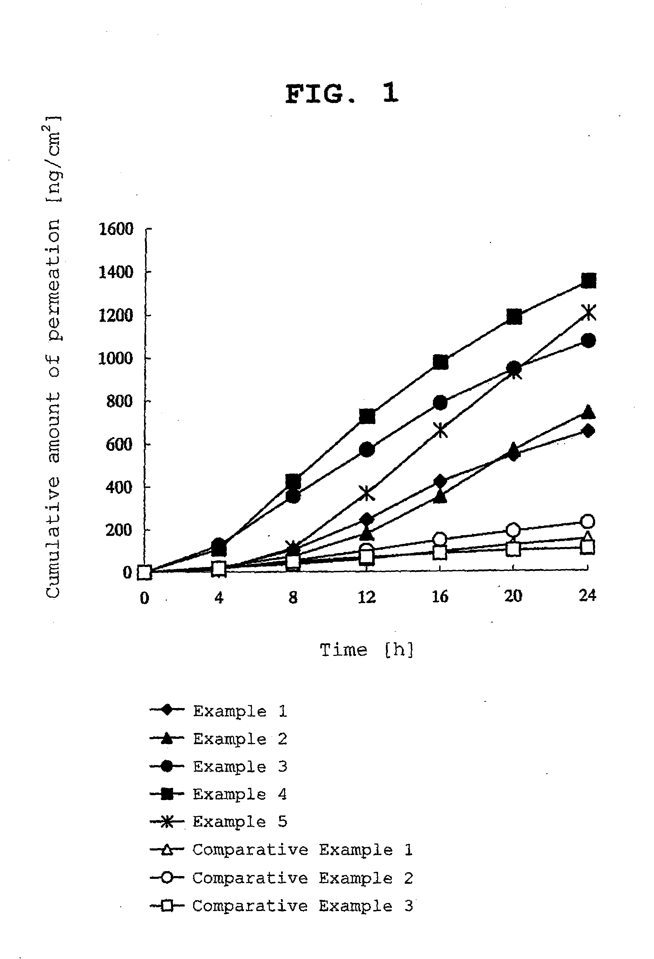

Fig. 1 shows the time-course changes in the cumulative amount of permeated capsaicin in each Example and Comparative Example.

### EFFECT OF THE INVENTION

The tape preparation of the present invention comprises a support and an adhesive layer, wherein capsaicin as a percutaneous absorption drug is contained in the adhesive layer containing a rubber adhesive containing at least one kind of rubber polymer. Since the adhesive layer has an appropriate adhesive force while maintaining cohesion, it shows extremely fine adhesive property and superior capsaicin releaseability, and can afford a rapid pharmacological effect with a small capsaicin content.

### BEST MODE FOR CARRYING OUT THE INVENTION

While the support to be used for the tape preparation of the present invention is not particularly limited, plastic films of polyethylene, polypropylene, polyester, polyvinyl acetate, ethylene-vinyl acetate copolymer, polyvinyl chloride, polyurethane and the like, metal foils such as aluminum foil, tin foil and the like, non-woven fabric, woven fabric, knitted fabric, paper and the like can be used independently or in the form of a lamination film thereof and the like. Since promotion of transdermal transfer of drugs can be expected due to the effect of occlusive dressing technique (ODT effect), a PET film and the like are preferably used. To reduce irritation to the skin during adhesion, flexible knit and the like superior in the followability to the skin are also preferable, and a laminate of a PET film and knit and the like can also be used.

The thickness of the above-mentioned support is not particularly limited as long as it has a thickness capable of affording mechanical strength of the level free of uncomfortableness by adhesion of the tape preparation of the present invention to the skin surface, and breakage when peeled off from the skin surface, and is generally about 1-2000 µm, preferably 1-1000 µm.

In the tape preparation of the present invention, the adhesive layer to be formed on at least one surface of the above-mentioned support comprises capsaicin and a rubber adhesive, and has appropriate skin adhesiveness and cohesion.

As a rubber adhesive, one comprising a high molecular weight rubber polymer having a viscosity average molecular weight of 150,000-4,000,000 is preferably used. The high molecular weight rubber polymer is essential for imparting a rubber adhesive with appropriate cohesion, and preferably contained in the rubber adhesive in a proportion of not less than 10 wt%, more preferably not less than 20 wt%. Examples of the high molecular weight rubber polymer include polyisobutylene, polyisoprene, polybutadiene, styrene-isoprene-styrene block copolymer, styrene-butadiene-styrene block copolymer and the like, which can be used as a mixture of one or more kinds thereof.

The rubber adhesive preferably contains a high molecular weight polyisobutylene having a viscosity average molecular weight of 150,000-4,000,000, more preferably 800,000-3,000,000, and preferably further contains, for the purpose of controlling the adhesiveness, a middle molecular weight polyisobutylene having a viscosity average molecular weight of 10,000-150,000, more preferably 40,000-90,000 and/or a low molecular weight polyisobutylene or polybutene having a viscosity average molecular weight of 500-4,000. Here, it is preferable to contain a high molecular weight polyisobutylene in a proportion of 10-80 wt% (preferably 20-70 wt%, particularly preferably 40-60 wt%), a middle molecular weight polyisobutylene in a proportion of 0-90 wt% (preferably 10-80 wt%, particularly preferably 20-50 wt%), and a low molecular weight polyisobutylene in a proportion of 0-80 wt% (preferably 10-60 wt%).

When the content of the high molecular weight rubber polymer is lower than the above-mentioned range, cohesion is degraded, and adhesive deposit may occur during adhesion. When the content exceeds the above-mentioned range, the adhesive may become too hard to cause an adhesion failure.

The viscosity average molecular weight in the present invention is calculated by the Flory's formula.

To impart appropriate adhesiveness to the rubber adhesive, for example, one or more kinds of tackifiers such as rosin resin, polyterpene resin, chroman-inden resin, petroleum resin, terpene-phenol resin, xylene resin, alicyclic saturated hydrocarbon resin and the like may be added. The content of the tackifier is preferably not more than 50 wt%, particularly 5-40 wt%, more preferably 10-30 wt%. When the content exceeds 50 wt%, the adhesiveness becomes too strong to cause pain during peeling off, exfoliation of the keratin layer and irritation to the skin.

It is also possible to add liquid fatty acid ester to an adhesive layer at room temperature to allow it to be compatiblized with the above-mentioned rubber adhesive and uniformly distributed in the adhesive layer. As a result, these components plasticize the adhesive layer, which softens the adhesive layer and reduces pain and skin irritation felt due to the skin adhesiveness when peeling off the tape preparation of the present invention from the skin surface. Moreover, since the adhesive layer is plasticized as mentioned above, capsaicin contained therein has better free diffusibility, which improves release (transdermal transfer) to the skin surface.

Therefore, when a fatty acid ester is used in the present invention, the fatty acid ester only needs to plasticize the adhesive layer. When the fatty acid ester comprises a fatty acid having an unnecessarily large or small carbon number, the compatibility with the aforementioned rubber adhesive may be degraded, or the fatty acid ester may be volatilized in a heating step during production of the preparation. In addition, a fatty acid ester comprising an unsaturated fatty acid having a double bond in a molecule may cause oxidative degradation and the like, which may impair preservation stability.

Therefore, as the fatty acid ester to be used in the present invention, a higher fatty acid preferably having 10 to 16, more preferably 10 to 14, carbon atoms and a lower monovalent alcohol having 1 to 4 carbon atoms is employed. As such higher fatty acid, preferred are decane acid (C10), lauric acid (C12), myristic acid (C14) and palmitic acid (C16). As the lower monovalent alcohol, methyl alcohol, ethyl alcohol, propyl alcohol and butyl alcohol can be mentioned, which are not limited to straight chain alcohols but may be branched alcohols. As preferred fatty acid ester, isopropyl myristate, methyl decanoate and the like can be mentioned.

When the above-mentioned fatty acid ester is used for the tape preparation of the present invention, the content of the fatty acid ester is set to 10-120 parts by weight, preferably 20-100 parts by weight, relative to 100 parts by weight of the rubber adhesive.

When the content of the fatty acid ester is less than the above-mentioned lower limit, a sufficient effect of addition cannot be achieved. When the content exceeds the above-mentioned upper limit, the fatty acid ester may not be maintained in an adhesive, and the adhesive component and the fatty acid ester may be separated (blooming) in the surface of the adhesive to degrade the skin adhesiveness.

The tape preparation of the present invention comprises capsaicin in an adhesive layer as a percutaneous absorption drug. While the content of capsaicin can be appropriately set according to the administration object and subject of administration, it is generally 0.005 - 0.05 wt%, preferably 0.01 - 0.04 wt%, of the adhesive layer. When the content is less than 0.005 wt%, transdermal transfer of a therapeutically effective amount of capsaicin cannot be expected, and when the content exceeds 0.05 wt%, a strong initial stimulant pain is caused by capsaicin.

The thickness (thickness after drying) of the above-mentioned adhesive layer is appropriately determined according to the application site, an adhesive to be used and the like, and it is generally about 10 µm - 200 µm, preferably about 15 - 150 µm.

The tape preparation of the present invention is produced by forming an adhesive layer containing an adhesive, a drug and, where necessary, a tackifier on a support. The method for forming an adhesive layer on a support is not particularly limited and known production methods of tape preparation can be employed. For example, a solution of the aforementioned adhesive is applied onto a release liner after a surface release treatment to form an adhesive layer having a desired thickness, the solvent is evaporated and a support is laminated. Alternatively, a solution of the aforementioned adhesive is applied onto a support to form an adhesive layer having a desired thickness, the solvent is evaporated and a release liner after a surface release treatment is laminated.

As a coating method of an adhesive solution, the methods conventionally used can be generally employed. For example, cast method, roll coater method, reverse coater method, doctor blade method, bar coater method and the like can be mentioned.

In the present invention, the above-mentioned adhesive layer is formed directly or indirectly, via a primer for improving anchor property and the like, on at least one surface of a support, in view of the handling property, adhesion to the skin and the like.

The tape preparation of the present invention has an analgesic or antipruritic effect based on capsaicin, and its indication includes postherpetic neuralgia, diabetic neuralgia, essential pruritus, psoriasis, cluster headache, post-mastectomy pain syndrome, stomatitis, skin allergy, urination reflection facilitation, lumbago in hematuria syndrome, cervical pain, stump pain, reflex sympathetic atrophy, pain due to skin cancer, arthritis and the like.

### Examples

The present invention is explained in detail in the following by referring to Examples, which are not to be construed as limitative. In the following, "part" and "%" mean "parts by weight" and "wt%", respectively.

### Example 1

High molecular weight polyisobutylene (52 parts, viscosity average molecular weight 2,100,000, product name: VISTANEX MML-140, manufactured by Exxon chemical Co.), middle molecular weight polyisobutylene (28 parts, viscosity average molecular weight 60,000, product name: HIMOL 6H, manufactured by NIPPON PETROCHEMICALS CO., LTD.) and alicyclic petroleum resin (20 parts, softening point 100°C, product name: ARKON P-100, manufactured by Arakawa Chemical Industries, Ltd.) were dissolved in hexane to give a rubber adhesive solution. To this solution was added capsaicin such that its content in the adhesive layer became 0.025%. The mixture was thoroughly stirred and applied onto a release liner such that the thickness after drying became 80 µm. The mixture was dried to give an adhesive layer. A polyester knit support (basis weight 107 g/m², thickness 500 µm) was laminated on the adhesive layer to give a tape preparation of the present invention.

### Example 2

In the same manner as in Example 1, a rubber adhesive solution was prepared, and isopropyl myristate (fatty acid ester) and capsaicin were added to an adhesive layer such that their contents in the adhesive layer were 40% and 0.025%, respectively. The mixture was thoroughly stirred and, in the same manner as in Example 1, a tape preparation was obtained.

### Example 3

In the same manner as in Example 2 except that the support was changed to a PET film (thickness 25 µm), the tape preparation of the present invention was obtained.

### Example 4

In the same manner as in Example 2 except that the fatty acid ester was changed to methyl decanoate, the tape preparation of the present invention was obtained.

### Example 5

A styrene-isoprene-styrene block copolymer (40 parts, weight average molecular weight 200,000 (GPC vs polystyrene standard), viscosity 490 mPa·S (B type viscometer, polymer concentration 25 wt%, toluene solution), product name: Quintac 3450, manufactured by ZEON CORPORATION), a low molecular weight polybutene (30 parts, viscosity average molecular weight 1,260, product name HV-300, manufactured by NIPPON PETROCHEMICALS CO., LTD.) and an alicyclic petroleum resin (30 parts, softening point 100°C, product name ARKON P-100, manufactured by Arakawa Chemical Industries, Ltd.) were dissolved in toluene to give a rubber adhesive solution. Capsaicin was added to this solution such that its content in the adhesive layer was 0.025%. The mixture was thoroughly stirred and, in the same manner as in Example 1, a tape preparation was obtained.

### Comparative Example 1

2-Ethylhexyl acrylate (95 parts) and acrylic acid (5 parts) were polymerized in ethyl acetate under an inert gas atmosphere to give an acrylic adhesive solution. Isopropyl myristate (fatty acid ester), capsaicin and trifunctional isocyanate as a crosslinking agent (product name: CORONATE HL, manufactured by NIPPON POLYURETHANE INDUSTRY CO., LTD.) were added to this solution such that their contents in the adhesive layer were 40 %, 0.025 % and 0.09 %, respectively. The mixture was thoroughly stirred and applied onto a release liner such that the thickness after drying became 80 µm. The mixture was dried to give an adhesive layer. A polyester knit support (basis weight 107 g/m², thickness 500 µm) was laminated on the adhesive layer and the laminate was aged at 60°C for 24 hr to give a tape preparation of the present invention.

### Comparative Example 2

2-Ethylhexyl acrylate (75 parts), N-vinylpyrrolidone (22 parts) and acrylic acid (3 parts) were polymerized in ethyl acetate under an inert gas atmosphere to give an acrylic adhesive solution. Isopropyl myristate (fatty acid ester), capsaicin and trifunctional isocyanate as a crosslinking agent (product name: CORONATE HL, manufactured by NIPPON POLYURETHANE INDUSTRY CO., LTD.) were added to this solution such that their contents in the adhesive layer were 40 %, 0.025 % and 0.09 %, respectively. The mixture was thoroughly, stirred and, in the same manner as in Comparative Example 1, a tape preparation was obtained.

### Comparative Example 3

A hydrophilic ointment was prepared according to the method described in the Japanese Pharmacopoeia. To this hydrophilic ointment was added a 10% ethanol solution of capsaicin in a proportion of 0.25%, i.e., capsaicin content of the preparation of 0.025%. This was thoroughly kneaded to give an ointment preparation (Hospital Pharmacists Preparation, Fourth Edition, Japan Hospital Pharmacists Association ed., 189. 1997).

### Experimental Example 1

Adhesive property: The tape preparations (10 cm²) obtained in Examples 1 - 5 were adhered to the chest of 8 healthy volunteers for 8 hr, and adhesive property was evaluated based on the presence or absence of turning up during adhesion, pain during peeling off, and partial remainder of adhesive layer on the skin after peeling off or adhesive deposit. As a result, none of the tape preparations showed turning up during adhesion. No pain was reported during peeling off, nor was there observed adhesive deposit. Thus, all tape preparations of the present invention had superior adhesive property.

### Experimental Example 2

Snake exuviae permeability: The tape preparations obtained in Examples 1-5 and Comparative Examples 1 and 2 were punched out in 9 mm_{φ}, and adhered to the center of 18 mm_{φ} African rock python exuviae. In addition, the ointment preparation (5.1 mg, containing the same amount of capsaicin as in a tape preparation) obtained in Comparative Example 3 was uniformly applied to the center of 18 mm_{φ} snake exuviae and covered with a polyester knit. These snake exuviae were set on a permeation testing apparatus (Automatic Flow Thru Diffusion Cell Apparatus, manufactured by Vangard International Inc.), and the amount of capsaicin permeated into water on the receptor side was measured by liquid chromatography. The results are shown in Fig. 1 (n=3) as the time-course changes in the cumulative amounts of permeation of capsaicin.

As is clear from Fig. 1, all the tape preparations of the present invention were superior in the release of capsaicin.

## Claims

1. A tape preparation comprising a support and an adhesive layer directly or indirectly formed on at least one surface of the support, wherein the adhesive layer comprises a rubber adhesive containing at least one kind of rubber polymer and capsaicin as an active ingredient.

2. The tape preparation of claim 1, wherein the rubber adhesive further comprises a tackifier.

3. The tape preparation of claim 1, wherein the rubber adhesive layer further comprises a fatty acid ester.

4. The tape preparation of any of claims 1 to 3, wherein the fatty acid ester is obtained from a higher fatty acid having 10 to 16 carbon atoms and a lower monovalent alcohol having 1 to 4 carbon atoms.

5. The tape preparation of any of claims 1 to 4, which is used for the treatment of pain or itching.

6. The tape preparation of claim 5, wherein the pain or itching is associated with postherpetic neuralgia, diabetic neuralgia, essential pruritus, psoriasis, cluster headache, post-mastectomy pain syndrome, stomatitis, skin allergy, urination reflection facilitation, lumbago in hematuria syndrome, cervical pain, stump pain, reflex sympathetic atrophy, pain due to skin cancer or arthritis.
